# EUROPEAN PATENT SPECIFICATION

(11) **EP 4 591 907 B1**
(45) Date of publication and mention of the grant of the patent: **24.06.2026**
(21) Application number: 25152666.1
(22) Date of filing: 17.01.2025
(51) Int. Cl.: A61M 11/02, A61M 15/00, A61M 15/08

(54) **MULTI-CHAMBERED POWDER DISPENSE SYSTEM**
MEHRKAMMER-PULVERABGABESYSTEM
SYSTÈME DE DISTRIBUTION DE POUDRE À CHAMBRES MULTIPLES

(30) Priority: 23.01.2024 US 202463623926 P
(43) Date of publication of application: 30.07.2025
(73) Proprietor: Zeteo Biomedical LLC, Austin, TX 78738 (US)
(72) Inventor: GJERTSEN, Jeff, Cedar Park (US); SULLIVAN, Timothy, Austin (US)
(74) Representative: FRKelly

(56) References cited:
- EP-A2- 1 106 196
- WO-A2-2004/093848
- GB-A- 2 340 758
- US-A1- 2009 320 837
- US-A1- 2011 277 763
- US-A1- 2023 293 831
- US-B2- 10 688 260
- US-B2- 8 834 411

## Description

### CROSS REFERENCE TO RELATED APPLICATIONS

This application claims priority and benefit from U.S. Provisional Patent Application No. 63/623,926 filed on January 23, 2024, entitled "Multi-Chambered Powder Dispense System,".

### BACKGROUND OF THE INVENTION

### TECHNICAL FIELD

Embodiments of the subject matter disclosed herein generally relate to medical devices. More particularly, the present invention is in the technical field of medical devices for the administration of medicinal compounds as powders to humans or other mammals.

### DISCUSSION OF THE BACKGROUND

Certain diseases and medical conditions that are systemic or local are treatable, including prophylactically, via the administration of drugs and therapeutic agents taken topically or systemically through the eye, ear, mouth, nose, lungs or dermal skin layer. There are a growing number of medicaments that are most effectively manufactured, stored, delivered, and administered as a dry powder formulation. A number of pharmaceutical agents are deliverable as powders or particles orally to the lungs, sublingual, buccal or intra-nasally (including nose to brain), and may be administered for topical, systemic or intracranial deposition, including but not limited to antibiotics, antipyretics, anti-inflammatories, biologics, biosimilars, vitamins, botanicals, co-factors, enzymes, inhibitors, activators, nutrients, vaccines including DNA based killed or live virus or microorganisms, nucleic acids, proteins, peptides, antibodies, peptide mimetics, prophylactic or therapeutic immune-modulators, anti-viral and anti-bacterial compounds, diagnostic agents and other agents, pharmaceutical compositions or medicaments.

Solid formulated medicaments have a number of recognized advantages. Compound stability for certain agents is greater in solid form especially polypeptide and protein based biologics whose conformational and higher structure may tend to degrade or denature when in solution thus affecting their biological activity. Similarly, certain drug chemical compounds may tend to dissociate and degrade due to incremental pH shifts, Van der Waals and other forces resulting in diminished shelf life and drug efficacy. Consequently, unstable medicaments formulated as liquids must be refrigerated or even frozen to preserve their potency or effectiveness which adds cost and complicates deployment. This is especially troublesome in such cases whereby vaccines and other unstable medicaments must be distributed to remote areas and underdeveloped regions or very rapidly to large populations during a public health crisis under austere field conditions. Often unstable drugs must then be shipped in solid form and reconstituted back to liquid form at the time of administration thus delaying deployment and adding expense and the need for skilled personnel for proper utilization.

In certain other cases medicaments are designed in solid form to facilitate controlled release to result in sustained pharmacological concentrations of active ingredients over an extended period of time. For systemic treatments, powder based drugs delivered to mucosal surfaces via the nose, lungs or oral cavity offer a number of advantages including rapid drug uptake due to large mucosal surface area capable of systemic uptake, the avoidance of the harsh environment of the stomach and intestinal tract as in the case of pills, tablets, and capsules, and the avoidance of broad systemic and side effects often associated with parenterally administered drugs. Other advantages include enhanced bioavailability, reduced dose volume, rapid onset of therapeutic response, improved patient compliance and ease of self-administration. Powders can be formulated and dispensed to deliver medications topically to wounds, into the nose or mouth to reach the upper respiratory tract for the treatment of a localized condition or as a prophylactic.

Typically, these agents and medicaments are formulated and prepared from solution by recrystallization followed by milling, but for improved control over particle crystallinity, shape, mean size, and size distribution; lyophilization or various spray drying techniques known in the art are relied upon to produce a bulk powder with precise characteristics to aid in administration. Key characteristics include primarily the mean particle size as well as the distribution of sizes within the bulk powder. For a given inspiratory velocity initiated either nasally or orally, a certain mean particle size and mass is required to result in deposition to the targeted tissue location within the targeted area within the respiratory tract. Generally, smaller particles will tend to deposit deeper in the respiratory tract, more particularly; particles of 3 or fewer microns in diameter have a greater probability to reach the tissues of the lower lungs, with even smaller aerodynamic diameters preferred for enhanced systemic uptake. Conversely, larger particles of greater than 5 microns to the tens of microns or larger, owing to their larger mass are more likely to deposit proximally to the point of administration; most typically within the nasal cavity and passages when administered intranasally, or in the oral cavity or pharynx, larynx, or trachea if orally administered. The dispersity or polydispersity index describes the range and proportion of sizes within the bulk powder. Depending upon the targeted application location, a less disperse or mono-disperse powder may be desired to assure a specific deposition location or a more disperse powder may be necessary in order to impact a larger range of tissues such as the case with certain anti-viral therapies and vaccines where the intent is to contact the virus residing in several tissue areas and locations within the upper and lower respiratory tract.

Other aspects of powder engineering are intended to impact the flowability, absorption efficiency and reduce the aggregation of the powders in order to aid in the friability of the material to increase the delivery efficiency, efficacy and rate of uptake. For that reason, certain excipients, carriers, or other matrix components may be added in defined quantity to the active dry pharmaceutical agent to impact particle shape, texture and surface properties for reduced adhesive and electrostatic forces in order to facilitate the breaking apart of settled or aggregated particles prior to and during dispense. Other excipients, carriers, or other matrix components may be added in defined quantity to the active dry pharmaceutical agent to impact mucosal absorption or dwell time on the targeted deposition site. Further, micro and nano particle formulations of drugs are often employed using biocompatible and degradable polymers as carriers.

All of these and other powder engineering principles play an important role in conjunction with the design of packaging and dispensing systems and devices to achieve precise delivery and dispense characteristics of powdered drugs. A variety of packaging and devices are known for delivering a controlled quantity of a dry pharmaceutical preparation to the ear, dermis, nose, nasal and olfactory mucosa, sublingual, buccal, oral mucosa, pharyngeal, tracheal, and lower respiratory tissues.

Unlike liquid drug formulations, whereby a simple pump can deliver a precisely controlled quantity of drug as droplets with the required spray characteristics; drugs formulated as dry materials present additional challenges owing to the propensity of powders to settle and physically and chemically agglomerate. Thus, it is necessary that the device must not only contain a single dose of material or be capable of metering it from a bulk source but must also impart sufficient energy to agitate the material to break up the particles and propel them to the deposition site in the correct quantity and mean particle size in order to provide optimum deposition characteristics, and consequently the most advantageous therapeutic effect.

There exists numerous systems and devices to dispense powders to a human or non-human subject; the basic designs of which vary depending upon the site of administration, target deposition zone and intended topical, systemic or intracranial application. For example, Dry Powder Inhalers (DPIs) is the class of devices that is a common type of device for delivering dry pharmaceutical preparations to a user most typically for pulmonary deposition via oral administration. Typically, such devices utilize an external propellant, pressurizer or other external energy source which classifies those devices generally as Active Dry Powder Inhalers (ADPIs) and offer the purported benefit of more precise dosing. Alternatively, other devices rely solely on the inspiratory airflow of the user and hence are breath actuated and referred to as Passive Dry Powder Inhalers (PDPIs). Both approaches suffer from significant drawbacks. In the case of ADPIs, owing to the need for a propellant or electromechanical componentry and often bulk storage of drug; the device itself can be complex, large, expensive, cumbersome, and inconvenient to handle and use. Passive devices while often smaller, less expensive and containing one or more individualized unit doses; often deliver inconsistent quantity of drug to the user with the variability of delivered dose a function of user inspiratory flowrate. Further, the passive devices often operate at a reduced efficiency as given by the fraction of the dose quantity actually delivered to the user. The reduced efficiency diminishes the cost effectiveness of the passive devices due to wasted drug material. The undispensed portion of the drug that remains is also left behind to contaminate the device, and in the case of multi-dose devices, possibly contaminate subsequent doses of drug.

In the case of pulmonary deposition, very small particles (1-5 microns) are preferred but smaller particles typically suffer from an increased tendency to form clumps due to hygroscopicity, adhesion and electrostatic forces. Prior art devices commonly rely on high velocity propellants or electromechanical agitation to de-aggregate the powder particles and deliver the material to the target deposition zone of the user. The means of providing the external energy source are widely varied and include pressurized canisters, propeller type agitators, mechanical, solenoid or piezoelectric based vibration to aid in particle deaggregation and delivery. For example, Gumaste in U.S. Patent No. 7,950,390 discloses a microelectronic piezo vibrator to aid breaking apart the agglomerated particles and suspending them into the flow field. Such microelectronic systems offer improvements in the bulk size of the device as compared to Wilke et. al., who in U.S. Patent No. 3,948,264 discloses a battery driven electro-mechanical vibrator to facilitate dispersion and release of the particles. These schemes, while incrementally different, consistently suffer from the disadvantage of system complexity due to the need for circuitry, motors, and electrical power sourcing. Additionally, these prior art devices often entail capsule based dosage forms externally pierced by various means often including retractable mechanical or motor driven pins, often arranged in multiple pin arrays and channels to facilitate increasing the fraction ejected from the dosage form.

Alternatively, passive devices rely upon the forceful inhalation by the user to disperse the particles and deliver them to the airway and the targeted tissues. In most prior art active and passive devices, the operation often entails a series of steps to facilitate administration of drug. Additionally, the dosage forms are often singularized into the form of capsules containing the prescribed dose quantity that must first be externally pierced in order to expose the compound to the velocity field. The other dosage form common in such devices are individual blisters either singularized or in strips or cartridges that are loaded into the dispensing device and also first require either piercing of the blister or peeling of the upper lidding layer to expose the contents. For example, Davies et al in U.S. Patent Nos. 5,590,645; 5,860,419; 5,873,360; 6,032,666 discloses an inhalation device with a multi-dosage configuration in the form of a strip of individual blisters containing the medicament. The base and lid materials are peeled apart as the strip is rotated into an opening station position and the two ends taken up on separate spools. Once in position and the contents exposed, the user then inhales the drug compound. This prior art device has the advantage of simplicity owing to the reliance upon the user's inhalation as the primary means of particle dispersion and delivery. However, that approach may result in poor dose consistency; as measured by patient-to-patient variability or dose to dose variability of an individual patient. This variability is a consequence of the natural range of possible patient inspiratory rates and velocities. Further, the passive scheme as disclosed whereby no means are provided to augment the ejection of the blisters, may result in incomplete dosing and low efficiency of delivery whereby medication is left in the blister. Further, the undelivered quantity continues to reside within the opened blister and once indexed may fall out into the device interior, contaminating both the device and possibly subsequent doses.

Various other devices within the prior art include measured quantities of dry powdered formulations and pharmaceutical compositions contained in a crushable ampoule, blister or other dosage form that entail forcing the form against an external piercing device during use, in order to pierce the dosage form and release the contents. These devices often still rely solely upon the inhalation force of the user to adequately break apart the settled and aggregated dose material into individual particles. Often such prior art devices incorporate various aspects on the exterior of the dosage form or in the device itself such as channels, variously configured inlets, outlets, and orifices or other turbulence promoting means for improving the dispersion of the particles. However, such schemes typically result in modest improvement in dose efficiency.

There is thus a need for powder delivery device that includes the advantages of an external propellant source made available by an internally pierced blister system that is simple, precise and user friendly. The present invention addresses these disadvantages in the prior art devices by providing for an internally pierced multi-chambered dosage form that can be expressed in a manner to provide improved powder dosage delivery efficiency and ejection fraction. Device technology has lagged current powder formulation and powder engineering capabilities such that the enhanced precision and effectiveness of new and existing powdered drugs can be fully harnessed. The present disclosure provides dosage forms with integrated dispense energetics integrated with an external propellant source for delivery of predetermined quantities of dry powder or granular pharmaceutical or medical compositions for local, intracranial and/or systemic action. Integrating the device energetics into the dosage form reduces overall device cost, complexity, and bulk to improve patient compliance and ease of use.

US10688260B2 discloses a multi-chambered well dosage form and device for single or multi dose capable for administration of pharmaceuticals of a range of particle sizes. The present dosage devices and systems provide for improved efficiency and patient ease of use owing to its compact size and simple design, low cost, and consistent, contamination free dosing of dry powder medicaments or other agents. The present disclosure provides a multi-chambered dosage form containing dry powder medical composition containing chamber well with an internal piercing device and one or more adjacent gas-filled chamber wells to aid the dispense of the powder contents to the user and in certain embodiments, without the requirement of an external energy sources common with active devices known in the art. Said methods, systems and devices provide increased ejection fraction and hence greater efficiency of drug delivery above that as provided by current devices.

### SUMMARY OF EXAMPLE EMBODIMENTS

The present disclosure provides a unit dosage form as detailed in claim 1, and a delivery system according to claim 9. Advantageous features are provided in dependent claims.

According to an embodiment, there is a unit dosage form for administration of a dry powder medicinal compound to a subject. The dosage form includes a first blister well containing a first internal piercing device; a second blister well containing a second internal piercing device; and a lidstock that forms a sealed interface separating the first blister well from the second blister well. The internal piercing devices include a base member and an elongated piercing tip with a channel extending from the base member. The first internal piercing device of the first blister well provides fluid communication with an external propellant source and the second internal piercing device of the second blister well provides the dry powder medicinal compound to the user.

By way of example, a piercing device internal to a dosage form for dispensing a medicinal compound to a user is herein described. The device includes a disk-shaped base member that seats within a blister well of the dosage form; an elongated piercer tip including an internal channel and a piercing tip; and a planar sealing surface formed from a widened section of the body of the elongated piercer tip and located above the base member and below the end of the piercing tip. The planar sealing surface creates a seal between the piercer and the underside of a lidstock of the blister well of the dosage form during expression.

According to yet another embodiment, there is a delivery system for administration of a dry powder medicinal compound to a subject. The delivery system includes a handheld dispensing device with more than one plunger;
a dosage form disposed within the handheld dispensing device including a first blister well containing a first internal piercing device, a second blister well containing a second internal piercing device, and a lidstock that forms a sealed interface separating the first blister well from the second blister well. A first plunger of the handheld dispensing device compresses the first blister well of the dosage form to provide fluid communication with an external propellant source and a second plunger is compresses the second blister well of the dosage form to provide the dry powder medicinal compound to the user.

### BRIEF DESCRIPTON OF THE DRAWINGS

The accompanying drawings, which are incorporated in and constitute a part of the specification, illustrate one or more embodiments and, together with the description, explain these embodiments. In the drawings:
**FIGS. 1A-1B** show two views of a multi-chambered dosage form according to an embodiment of the invention.
**FIGS. 2A-2B** show two exemplary internal piercing devices.
**FIGS. 3A-3B** show two views of another embodiment of an internal piercing device with a planar sealing surface.
**FIGS. 4A-4B** illustrates the operation of an internal piercing device with a planar sealing surface.
**FIG. 5** shows an exemplary handheld dispensing device and system, the system being according to an embodiment of the invention.
**FIGS. 6A-6C** shows the operation of the exemplary handheld dispensing device and system of Fig. 5.
**FIG. 7** shows an isometric view of an exemplary handheld dispensing device and system, the system being according to an embodiment of the invention.
**FIGS. 8A-8B** show isometric and 2D views of a handheld dispensing device and system, the system being according to another embodiment of the invention.

### DETAILED DESCRIPTION OF EXAMPLES OF THE INVENTION

The following description of the embodiments refers to the accompanying drawings, which comprise embodiments of the invention and comparative examples. The same reference numbers in different drawings identify the same or similar elements. The following detailed description is intended to provide enabling examples of the invention and its use and does not limit the invention. Instead, the scope of the invention is defined by the appended claims. The following embodiments are discussed, for simplicity, with regard to medical devices for administration of a medicament to a human subject, particularly medicaments in powder form administered intranasally to a human subject. However, the embodiments discussed herein are not limited to such elements as the invention may also enable powder based as well as non-powdered drug delivery to human and non-human subjects via any route of administration.

Reference throughout the specification to "one embodiment" or "an embodiment" means that a particular feature, structure, or characteristic described in connection with an embodiment is included in at least one embodiment of the subject matter disclosed. Thus, the appearance of the phrases "in one embodiment" or "in an embodiment" in various places throughout the specification is not necessarily referring to the same embodiment. The drawings are intended to be illustrative of the claimed features and unless stated otherwise are not to scale. Where a dimension of a given feature may be pertinent, the detailed description will indicate one or more examples of the range and units of said dimension where needed to enable the subject matter. Further, the described features, structures or characteristics may be combined in any suitable manner in one or more embodiments.

The present invention provides dosage forms of multi-chambered wells with at least one chambered well containing internal componentry which provides for piercing or opening of the form from the inside for dispensing of the drug to a user. Note herein that said dosage forms are commonly referred to in the art using alternative terms such as forms, units, unit dose or dosage forms, blisters, blister packs, blister wells, wells, chambered wells, ampoules, or similar terminology. The dosage forms described herein generally as "forms", "unit dosage forms", "wells", "blisters" or "chambered wells" etc. are thus used interchangeably and are intended to encompass the full scope of known formed receptacles commonly in use for pharmaceutical substance storage and delivery.

The dosage form can be generated using methods known to those of skill in the art, including, for example, form fill seal technology or blow fill seal technology, or by deep draw forming. The form-fill-seal process can be used to create a blister, for example a pack or strip of blisters, from rolls of flat sheet or film, filled with the dry powder pharmaceutically active agent, and closed or sealed on the same equipment. This process entails clamping the laminate or film material, pressing one or more forming pins through plates with appropriately sized and spaced openings to form the blister well or receptacle in which the pharmaceutically active agent, or an agent that may be mixed or combined with a pharmaceutically active agent is placed. The lidstock or lidding of the same or similar material is sealed across the blister well leaving a pierceable covering through which the agent is dispensed out of the blister.

For example, the crushable unit dosage forms of the present disclosure are blisters may be manufactured as described by Nelson in U.S. Pat. No. 7,963,089. The manufacturing processes for forming blister wells for unit-dose packaging in a continuous web can include a step of drawing a metal, polymer, or laminated metal-polymer foil or other suitable sheet of material with the appropriate mechanical characteristics to allow hot or cold forming and drawing known in the art. In certain embodiments, one or more plungers can be used to form a primary contour, the contour having a depth of at least 100% and up to 150% of the depth of the final formed recess or well. A second stage involves shaping the primary contour with one or more of the same or additional plunger(s) to the desired formed recess depth and shape, with a depth that is less than the depth of the primary contour, while substantially maintaining the surface area of the primary contour formed in the first stage. The contour or shape of the blister well can be formed to contain certain shape features, indentations, or be imparted with texture by the forming pins to provide for a means of securing the internal piercing device within the blister well or recess. The formed well or recess is then loaded aseptically with the predetermined quantity of sterile dry powder and the internal piercing device and a lidding material of the same or similar laminated material as the blister well or other sheeting material can be rolled atop the recesses and bonded to the well sheeting with adhesives, or by thermal or ultrasonic or other welding means. The mass and volume of particles dispensed from an individual blister are various depending upon the blister shape and volume, the required volume of headspace gas, and the powder characteristics, which are primarily the bulk density which is affected by the particle shape, size, and adhesion and aggregation properties, among others. For example, the solid dosage mass and volume for intranasal or orally administered pulmonary treatments may range from 1 to 50 milligrams or more. This is but a single typical range for one application; ranges for other indications and routes of administration and needed therapeutic quantities can vary substantially and are contemplated herein to include ranges to gram level masses and 1000 microliters liquid dose volume or more for certain topical administered compounds.

In certain embodiments, the individual blisters that can be formed in sheets are in later manufacturing steps, singulated into single doses that may comprise individual blisters or in preferred embodiments of the present disclosure, may include multi-chambered blister dosage forms. The blister is then typically installed into a single-use, disposable, non-reloadable device, or for use in devices which are reloadable with additional unit doses for subsequent dosing of the same or different patient(s). Alternatively, and depending upon the application and indication, the sheets may be formed and cut into rows, arrays, grids or other configurations of blisters suitable for use in multi-dose devices. Regardless of the shape, size, or geometric configuration of blisters, ampoules, or wells; each unit contains an internal piercing device.

The dosage forms may contain in certain embodiments a biologic, a biological agent, diagnostic agent, or a small or large molecule pharmaceutical drug or other compound. The drug dosage forms of the present disclosure are for use in delivery devices that deliver the drug compound as a dry powder, particles, granules or other agent or formulation as a dry material to a human or non-human animal. In certain preferred embodiments, the dosage forms described herein can be used, for example, to deliver one or more measured doses of a dry pharmaceutical, biologic or medical composition to the nasal passages, mouth, throat, trachea, pharynx, upper or lower airways to include into the lungs for the therapeutic or prophylactic treatment of local or systemic conditions.

Any powder or dry form pharmaceutical is contemplated by the present disclosure, including but not limited to antibiotics, antipyretics, anti-inflammatories, biologics, botanicals, probiotics, vitamins, co-factors, enzymes, inhibitors, activators, nutrients, aptamers, thioaptamers, anti-virals, immuno-modulators, diagnostic agents, vaccines including killed or live virus or microorganisms, nucleic acids, proteins, peptides, antibodies, peptide mimetics, micro or nanoparticles, or other agents known in the art. The following is a limited list of examples of general classes of medicaments administered through the nasal or oral cavity, ear or skin as dry powders for a host of indications which can include but not limited to anemia, asthma, bronchitis, rhinitis, flu, cancer, cystic fibrosis, diabetes, osteoporosis, hepatitis, arthritis, chronic or acute pain, immunodeficiency disorders, multiple sclerosis, endocrinological disorders, neurodegenerative disorders, ocular disorders, metabolic disorders, dermal disorders and wounds, etc. Drug compounds for treating those indications include various adjuvants, calcitonin, erythropoietin, heparin, inhibitors, insulin, interferons, interleukins, hormones, neurotropic agents, growth factors, stimulating factors, exosomes, vasodilators and constrictors, etc. This list is not intended to be exhaustive and in no way is inclusive of all possible conditions and diseases, drugs and compounds, or routes or targets of administration, but rather is to illustrate the breadth of dry powder drugs and indications employable in the present invention and contemplated by the present disclosure.

In certain embodiments, the medical compositions are in the form of a powder, or a dry pharmaceutical combined with one or more active agents and combinations of pharmaceutically acceptable carriers or materials to include matrix agents, diluents, preservatives, coatings, adsorption or absorption enhancing or delaying agents, excipients, salts, bulking or filling agents, anti-clumping agents, adjuvants, buffers, chelators, or other ingredients known to those in the art as needed to affect the drug's stability, flowability, adhesion, dispersion and deaggregation characteristics, or pharmacological uptake, efficacy, activity and rate of release. For example, in certain embodiments a predetermined quantity of biological or pharmaceutical material may be combined with mannose, lactose or other carrier or bulking agents known in the art. The drug may also be bound to or encapsulated within nanoparticles or other macromolecules to aid in stabilizing the drug and/or affecting the drug compound's rate of release over time. Any conventional media or agent compatible with the active agent is contemplated. More than one active agent may also be incorporated into the compositions, for the same or separate purposes. The phrase "pharmaceutically acceptable" refers to compounds and compositions that are appropriate for administration to humans or non-human animals.

The present disclosure provides various embodiments of crushable multi-chambered dosage forms that contain the dry powder as well as an internal piercing device that opens the dosage form and provides one or more communication channels for delivery of the powder from the blister to the user. The dosage form in certain embodiments also includes one or more additional internally pierced chamber wells to provide pressurization to the at least one powder containing well to aid in the expression of the powder via either manually or automated introduction of an external propellant.

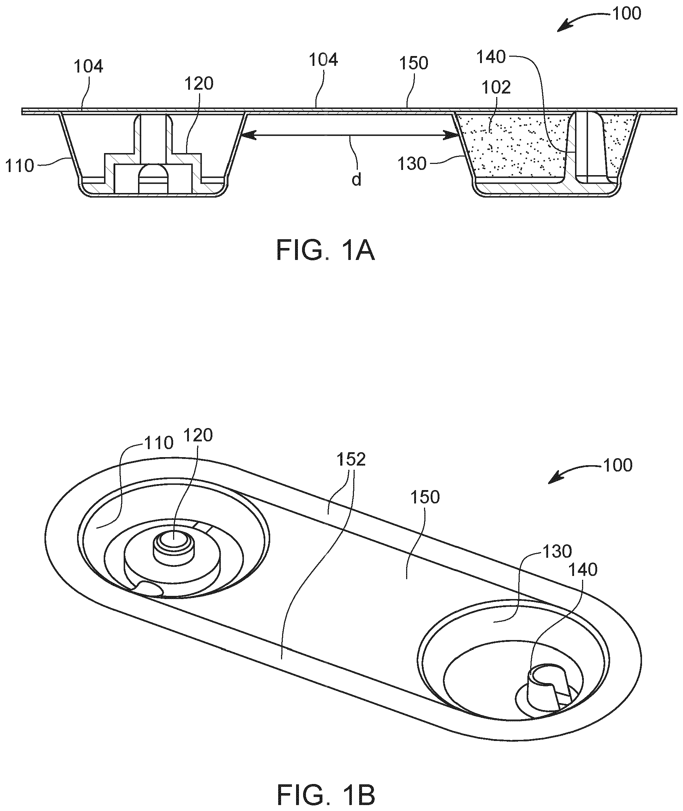

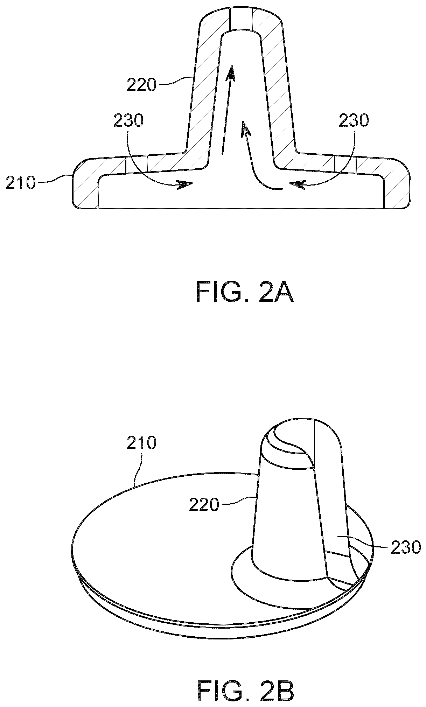

An exemplary multi-chambered blister dosage form assembly is illustrated in **Figs. 1A and 1B****.** In the embodiment shown in **Fig. 1A****,** blister assembly **100** includes two separate formed blister wells; a first blister well **110** and a second blister well **130.** The two wells are separated by a distance "d" with both wells covered by lidstock **104** which seals the perimeter **152** of the two wells as well as in between the two blisters thus segregating the contents of the two wells from one another until the dosage form is expressed. Upon pressurization of the blister wells during dispense, an inner portion of the sealed interface between the blister wells **110** and **130** and lidstock **104** separates while the perimeter **152** of the seal remains intact thus providing a fluid communication path **150** between the two blister wells as shown in top-down isometric view of **Fig. 2B** with lidstock **104** removed for clarity.

In certain preferred embodiments, each blister well **110/130** has a separate internal piercing device. In the dual chambered dosage form assembly **100** example, the first blister well **110** has a first internal piercing device **120** seated in its base, and the second blister well **130** has a second internal piercing device **140** similarly seated in its base, thus the two wells and piercers comprise a propellant blister and a dispense blister, respectively. The operation of dosage form **100** will be described in greater detail below, but briefly, the internal piercing device **120** of the first blister well **110** is configured to provide fluid communication to an external propellant source. Following activation of the propellant blister **110/120,** the dispense blister **130/140** is activated whereby its internal piercing device **140** causes the powder **102** formulated compound contained in the second blister well 130 to be administered to a user.

The internal piercing devices **120/140** are typically of polymeric composition and manufactured by techniques such as injection molding, machining or 3D printing. However, they may be constructed of any material with suitable chemical compatibility and mechanical properties to impart the design strength characteristics such as ceramic, glass, metal, composites, or other materials. In preferred embodiments the internal piercing device may be constructed from polymeric materials to include but not limited to polyethylene (PET), polypropylene, polystyrene, or poly ether ether ketone (PEEK), self-reinforced polyphenylene (SRP) or other pharmaceutical or medical grade material or materials. In preferred embodiments, the internal piercing devices may be molded, machined or printed as single piece components, however in certain other embodiments where certain structural features are less amenable to one-piece manufacture; the devices can be assembled from multiple machined and/or molded parts. For example, certain embodiments may entail attaching by snap fit or threading a machined metal elongated tip to a plastic base member. Other combinations of parts, manufacturing methods, materials, and assembly methods are fully contemplated herein.

When installed into a dosage blister form, and upon activation (e.g., crushing of the blister), the internal piercing device punctures the blister from the inside to provide fluid communication of materials into or out of the blister. Exemplary internal piercing devices are shown in **Figs. 2A-2B****.** A typical piercing device as shown in **Fig. 2A** comprises a base member **210** and an elongated piercer tip **220** with an internal channel **230** extending from the base member. Various structural features of an internal piercing device may be included, such as those described in U.S. Patents 8,834,411 and 10,688,260 which are co-owned by the present Applicant. Those features may include, for example, base member **210** composed of a flat disk (as shown) or a base ring which may be narrow, flat or tubular in shape. The base member **210** stabilizes the internal piercing device's position within the blister and provides a base for the elongated piercer tip **220.** The base member **210** may include connecting splines, spokes, or ribs of various shapes and numbers to connect the base member **210** to the elongated piercer tip **220.** Fluid communication into or out of the blister is provided by at least one channel **230** that in certain embodiments initiates in base member **210** and merges into the elongated tip's hollow center thus forming a single internal channel **230.** The hollow elongated piercer tip **220** may also include additional orifices, internal or cutaway type channels, as well as other structural features to affect agitation and turbulent flow within the blister and/or within the tip during piercing and dispensing of the dry powder or introduction of propellent. **Fig. 2B** depicts an embodiment of an internal piercing device which includes a flat disk-shaped base member **210** with an elongated piercer tip **220** that includes a cutaway or slot type channel **230** as contrasted to the internal channel **230** of **Fig. 2A****.** In this embodiment, cutaway channel **230** may provide a lessened propensity for a powder material to clog the elongated piercer tip as compared to fully internal channel beginning in the base member **210** and extending into the hollow elongated piercer tip as shown in **Fig 2A****.** Hence, an internal piercing device with an elongated piercer tip with a cutaway type channel **230** may, in certain embodiments, be preferably employed within the powder dispense blister **130/140,** while an internal channeled piercer tip may be employed in the propellent blister **110/120.** This combination of internal piercing device types corresponds to the dosage form embodiment as shown in **Figs. 1A-1B****.**

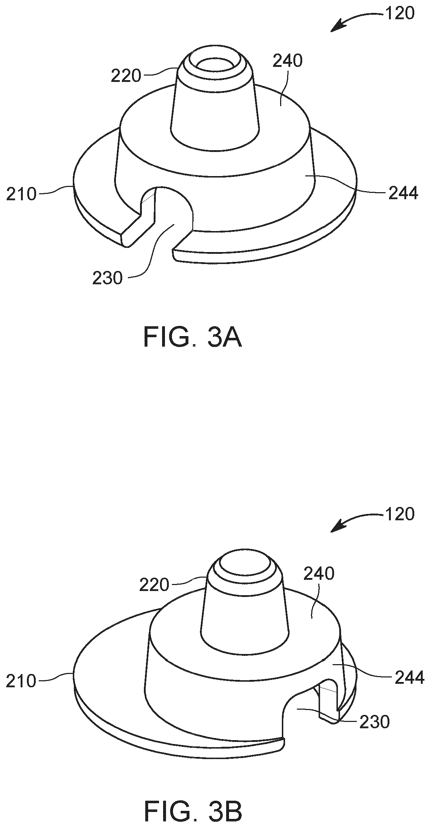

Also as shown in **Fig. 2B****,** the elongated piercer tip **220** of an internal piercing device may be positioned offset relative to the center of the base member **210.** Also, orienting the channel **230** axially such that the longitudinal axis of the channel is rotated upon the piercer base member **210** such that it is substantially opposite the side of the powder dispensing well **130/140** where propellant enters (see **Figs. 1A** and **1B**) may be advantageous. Such lateral positioning of the elongated piercer tip **210** and axial orientation of the channel **230** aids in providing a substantially unidirectional flow of propellant through the powder in the dispensing well. This arrangement generates a higher velocity region of flow very near the flow directing channel **230.** Acceleration of the propellant-powder mixture in the transition from the main region of the dispensing chamber **130** into the flow-directing channel **230** of the powder **140** piercing device provides improved agitation over known methods which more effectively de-agglomerates the powder. Note that though **Figs 3A-3B** depict as single channel **230,** in certain preferred embodiments, piercer **120** may include two channels **230** opposite one another or three channels **230** disposed at 60-degree intervals upon base member **210.** In the case of an embodiment whereby more than one channel **230** initiates in the base member **210,** each converges into a single channel within the body of a hollow elongated piercer tip **220.** Thus, in other embodiments, the same or different combinations of internal piercing types, with or without offset piercing tips, and/or with rotated or without non-rotated piercing tips may be employed in one or both blisters of the dosage form assembly **100** depending on the propellant and powder attributes and the desired dispense characteristics.

In another embodiment of internal piercing device **120** as shown in **Figs. 3A-3B****,** the device may further comprise a planar sealing surface **240** formed from a widened section **244** of the body of elongated piercer tip **220** with said planar sealing surface located above the base member **210** and below the end of the piercing tip. Planar sealing surface **240** thus provides a surface upon which a temporary seal is created between the piercer **120** and the underside of lidstock **104** of the propellant blister **110/120** during expression. Similar to other piercers described above, the elongated piercer tip **220** of internal piercing device **120** may also be positioned offset relative to the center of the base member **210** as shown in **Fig. 3B****.**

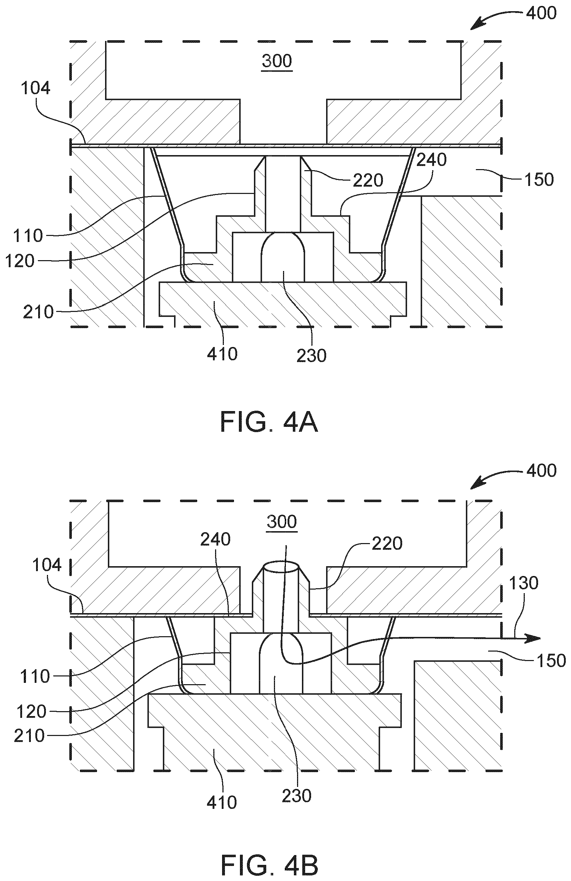

The basic operation of the planar sealing surface **240** in relation to the introduction of an external propellant source **300** into propellant blister **110** is depicted in **Figs. 4A-4B** as a partial view of a dispensing device **400** (to be discussed in greater detail below). As shown, a two chambered dosage form **100** including a first blister **110** as the propellant blister and a second blister **130** (not shown) as the dispense blister is loaded into a dispensing device **400.** An external propellant source **300** may be located anywhere in proximity to propellant blister **110,** but in this specific example, source **300** is located in contact with the lidstock **104** above the propellant blister **110** portion of dosage form **100.** When a first plunger **410** of dispensing device **400** compresses propellant blister **110** containing internal piercing device **120,** the elongated piercer tip **220** ruptures lidstock **104** thereby allowing fluid communication of the propellant **300** into blister **110** via at least one piercer channel **230.** Planar sealing surface **240** thus acts as a stop during blister compression and provides a seal between planar surface **240** and the underside of lidstock **104.** Without planar sealing surface **240,** increased compression of blister **110** may result in excessive travel such that lidstock **104** obstructs the one or more channels **230.** Propellant **300** is thus allowed to pressurize blister **110** which separates the lidstock from the base material of the propellant and dispense blisters thus opening fluid communication path **150** from propellant to dispense blisters remaining sealed along perimeter **152** (see **Figs 1A-1B**). As will be discussed in detail below, propellant **300** then pressurizes dispense blister **130** to dispense the powder to a user.

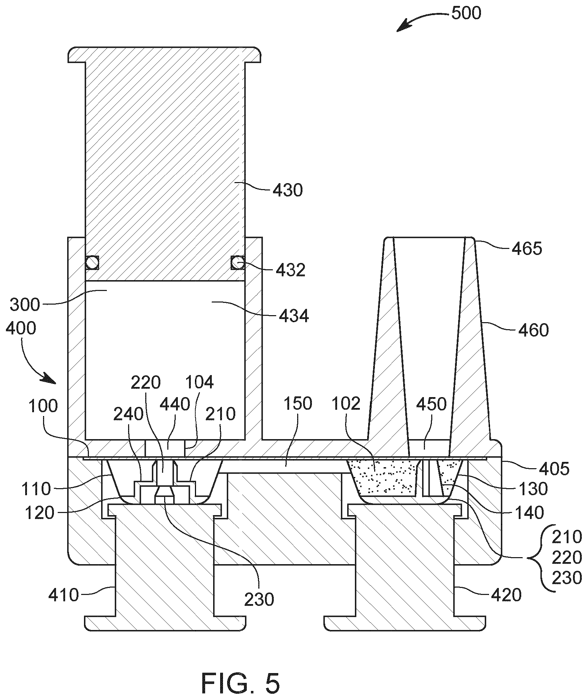

**Figure 5** shows an exemplary delivery system **500** for administration of a dry powder medicinal compound to a subject which includes a multi-chambered blister dosage form **100** loaded into a handheld dispensing device **400.** Dispensing device **400** may typically be composed of plastic or composite material that is molded, machined or 3D printed as a uni-body or multi-part assembly. In this example, device **400** includes housing **405** in a clamshell configuration suitable for insertion of a multi-chambered blister dose assembly **100,** but in other embodiments, housing **405** may include a slotted receptacle in one side for receiving assembly **100.**

Dosage form assembly **100** includes a propellant blister comprised of a first blister well **110** having a first internal piercing device **120,** and a dispensing blister comprised of a second blister well **130** having a second internal piercing device **140** with the two wells separated by a distance "d" and sealed by lidstock **104.** The body of dispensing device **400** is also configured such that a first plunger **410** and a second plunger **420** are provided at the base of the first propellant blister **110** and second dispensing blister **130** respectively to allow manual compression of the blisters during dispense. A propellant chamber **434** containing a propellant source **300** is positioned in this device example in contact with the lidstock **104** above propellant blister **110** and in preferred embodiments consists of air or other gas suitable for inspiration by a patient such as nitrogen. The propellant chamber in other embodiments may be positioned adjacent or otherwise within reasonable proximity to the dispensing device **400** whereby the propellant is in communication with propellant blister **110** via a tube or other conduit. In yet other embodiments, propellant source may be provided by a pre-compressed cylinder in communication with blister **110** and controlled by an electrically or manually controlled valve or restriction, etc. However, in the example shown in **Fig. 5****,** the device user introduces propellent **300** from chamber **434** by manual compression of a third plunger; the propellant chamber plunger **430.** Plunger **430** may thus further comprise O-ring **432** to maintain a seal against the inner wall of chamber **434** during compression. It should be noted that dispensing device **400** as shown may for illustrative purposes be described herein as a manually activated device or system embodiment, whereby the user manually activates the first thru third plungers (**410/420/430**). However, it is to be appreciated that any or a combination of the first, second or third plungers may be activated by electromechanical means which constitute additional embodiments contemplated herein.

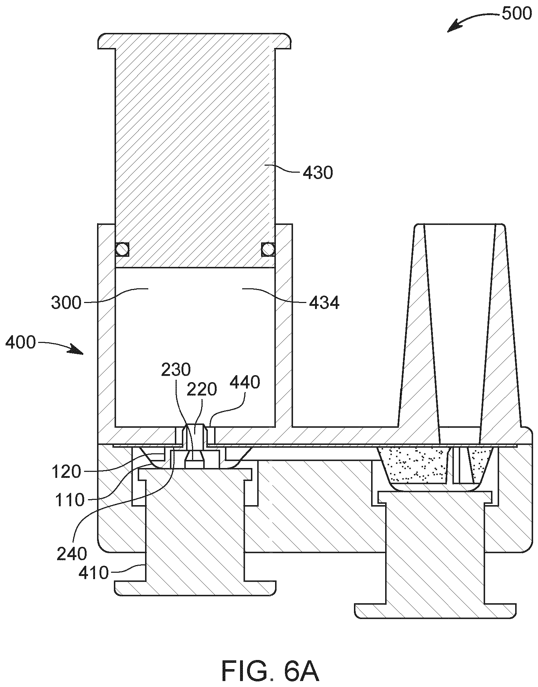

The operation of dispensing device **400** of delivery system **500** is shown in Figures **6A-6C****.** **Figure 6A** illustrates a first stage of dispense of the device in which a first plunger **410** compresses first propellant blister well **110** causing the elongated piercer tip **220** of the first internal piercing device **120** to puncture lidstock **104.** In this embodiment, internal piercing device **120** includes planar sealing surface **240** that acts as a stop during blister **110** compression and provides a seal between planar surface **240** and the underside of lidstock **104** (see also **Figs. 4A-4B**). Punctured lidstock **104** then allows for fluid communication between blister **110** and propellant chamber **434** via opening **440.** Propellant **300** may then flow into blister **110** via the at least one piercer channel **230** of internal piercing device **120.** Pressurization of propellant **300** may then be affected by compression of propellant plunger **430** by the user.

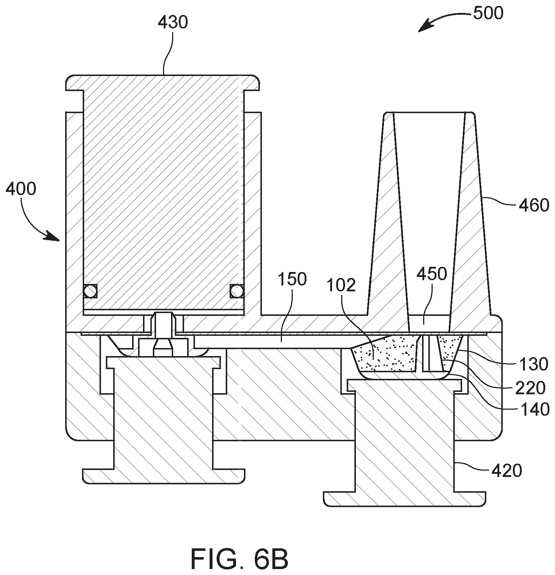

When propellant blister **110** is pressurized, lidstock **104** controllably separates from the multi-chambered blister well base material such that a fluid communication path **150** is opened while maintaining a seal around the perimeter **152** of dosage form **100** (see also, **Fig. 1B**). Propellant **300** then flows into the second (dispensing) blister well **130** which contains second internal piercing device **140** and the powder medicinal compound **102** as shown in **Fig. 6B****.**

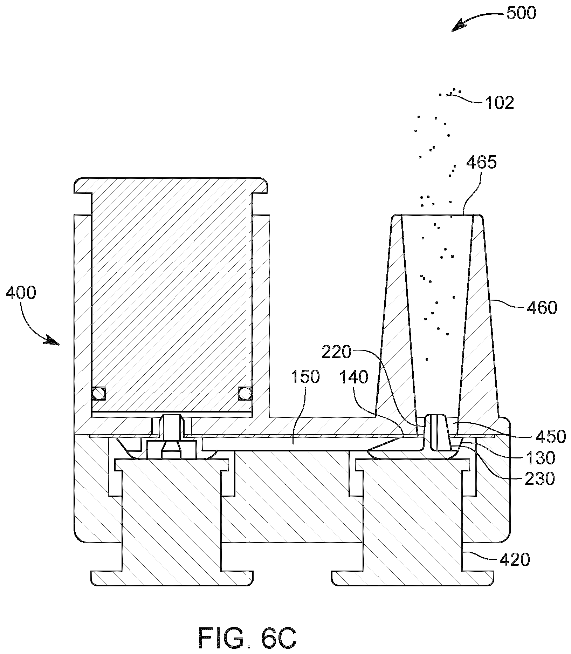

In a third stage of dispense, the dispense blister well **130** is compressed by the second blister well plunger **420** and the elongated piercer tip **220** of the second internal piercing device **140** punctures the lidstock **104** creating a pathway for powder **102** to enter into the dispense conduit **460** through opening **465** for administration to a user as shown in **Fig. 6C****.** It should be noted that powder **102** is in preferred embodiments contained solely in second dispensing blister well **130,** however, the first blister (propellant) well may also contain some portion of powder **102** as well. Also, in the embodiment shown, the elongated piercer tip **220** of second internal piercing device **140** is shown in a clocked and rotated configuration such that cutaway type channel **230** is rotated away from the entry of path **150.** This may aid in powder de-aggregation to improve powder ejection fraction, but as discussed above, other piercer configurations are contemplatable.

In certain other embodiments of the invention, the actions may occur in different sequence. For example, dispense blister well **130**'s plunger **420** may compress dispense blister **130** prior to the propellant blister **110** being partially or fully pressurized. In this approach, propellant **300** flows through the blister at a lower pressure, which may be advantageous depending upon the intended dose quantity as well as the characteristics of powder **102** for a given indication.

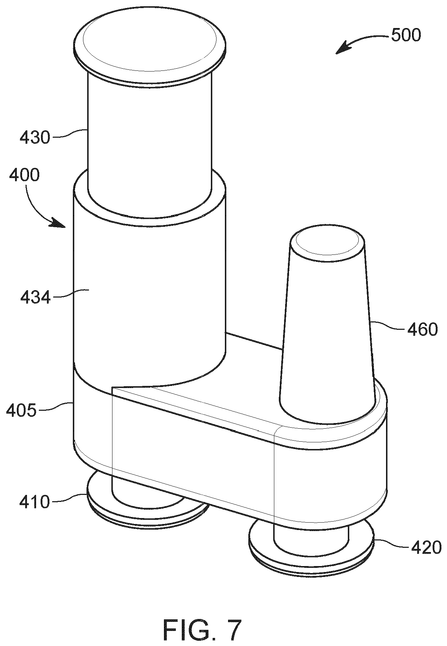

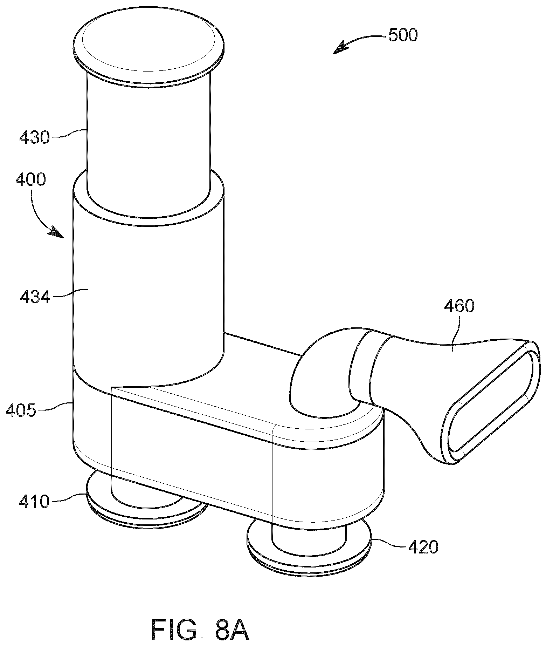

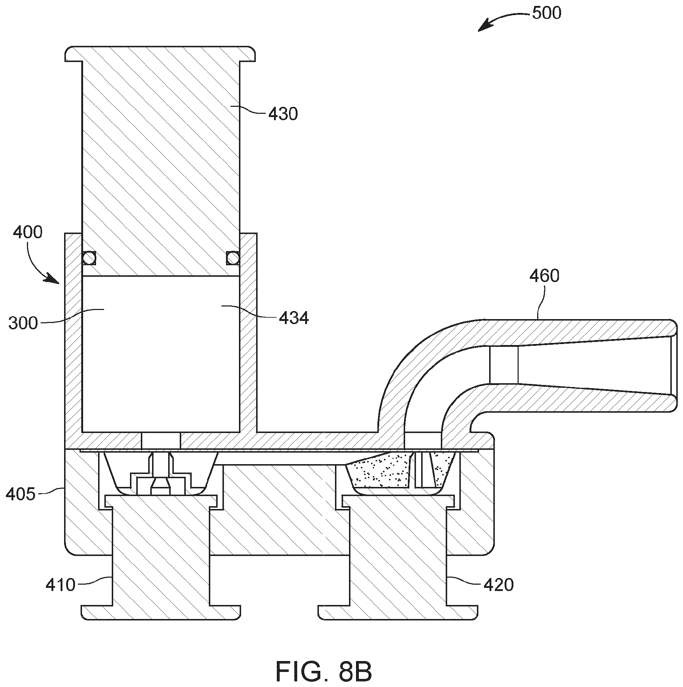

An isometric view of the delivery system **500** is shown in **Fig.** 7 which shows the housing **405** of dispensing device **400** along with first through third plungers **410/420/430** as well as propellant chamber **433** and dispense conduit **460** as configured generally for intranasal administration to a user. In other embodiments, dispensing device **400** of delivery system **500** may be configured for oral inhalation of the powder **102** for pulmonary delivery as shown in iso and 2D views of **Figs. 8A-****8B.** In this configuration, dispensing conduit **460** may include a shape that is more ergonomic for oral administration and may also include a bend to orient the conduit horizontally for ease of use by a user.

## Claims

1. A unit dosage form (100) for administration of a dry powder medicinal compound to a subject, the dosage form comprising:
a first blister well (110) containing a first internal piercing device (120);
a second blister well (130) containing a second internal piercing device (140);
a lidstock (104) that forms a sealed interface separating the first blister well from the second blister well;
wherein the internal piercing devices each comprise a base member (210) and an elongated piercing tip (220) with a channel (230) extending from the base member; and
wherein the first internal piercing device (120) of the first blister well is configured to provide fluid communication with an external propellant source and the second internal piercing device (140) of the second blister well is configured to provide the dry powder medicinal compound to the user.

2. The dosage form (100) of Claim 1, wherein the sealed interface separating the first blister well (110) from the second blister well (130) is configured to provide a fluid communication path (150) from the first blister well to the second blister well.

3. The dosage form (100) of Claim 1, wherein at least one of the internal piercing devices further comprises a planar sealing surface (240) located above the base member and below the end of the piercing tip.

4. The dosage form (100) of Claim 3, wherein the planar sealing surface (240) is located on the internal piercing device of the first blister well (110) configured to provide fluid communication with an external propellant source.

5. The dosage form (100) of Claim 1, wherein at least one of the internal piercing devices further comprises an elongated piercer tip (220) that is laterally offset relative to the center of its base member (210).

6. The dosage form (100) of Claim 1, wherein the elongated piercing tip (220) of at least one internal piercing device includes more than one channel (230) extending from the base member.

7. The dosage form (100) of Claim 1, wherein the channel (230) of the elongated piercer tip of at least one of the internal piercing devices is comprised of a cutaway type channel (230); and/or wherein the channel (230) of the elongated piercer tip of at least one of the internal piercing devices is rotatably configured on the base member (210) to provide a directional flow path.

8. The dosage form (100) of Claim 5, wherein the channel (230) of the internal piercing device is laterally offset relative to the center of its base member (210) and opposite the end of its blister well where propellant flows into the well during dispense.

9. A delivery system (500) for administration of a dry powder medicinal compound to a subject, the delivery system comprising:
a handheld dispensing device (400) comprising more than one plunger (410, 420, 430);
a dosage form (100) disposed within the handheld dispensing device comprising a first blister well (110) containing a first internal piercing device (120), a second blister well (130) containing a second internal piercing device (140), and a lidstock (104) that forms a sealed interface separating the first blister well from the second blister well; and
wherein a first plunger (410) of the handheld dispensing device is configured to compress the first blister well (110) of the dosage form to provide fluid communication with an external propellant source (434) and a second plunger (420) is configured to compress the second blister well (130) of the dosage form to provide the dry powder medicinal compound to the user.

10. The delivery system (500) of Claim 9, wherein the internal piercing devices (120, 140) each comprise a base member (210) and an elongated piercing tip (220) with a channel (230) extending from the base member.

11. The delivery system (500) of Claim 9, wherein the handheld dispensing device (400) further comprises a third plunger (430) to provide the external propellant to the first blister well (110); and/or wherein the handheld dispensing device further comprises an electromechanical device to provide the external propellant (300) to the first blister well; and/or wherein the handheld dispensing device (400) further comprises a mechanism to sequentially time the compression of the blister wells.

12. The delivery system of Claim 9, wherein the gas propellant (300) is nitrogen.

13. The delivery system (500) of Claim 9, wherein an elongated piercer tip (220) of at least one of the internal piercing devices further comprises a planar sealing surface (240) formed from a widened section of the body of the elongated piercer tip (220) located above the base member (210) and below the end of the piercing tip.

## Patentansprüche

1. Einheitsdosierungsform (100) zur Verabreichung einer trockenen, pulverförmigen Arzneimittelmischung an ein Subjekt, wobei die Dosierungsform Folgendes umfasst:
eine erste Blistervertiefung (110), die eine erste interne Durchstechvorrichtung (120) enthält;
eine zweite Blistervertiefung (130), die eine zweite interne Durchstechvorrichtung (140) enthält;
einen Foliendeckel (104), der eine abgedichtete Grenzfläche ausbildet, welche die erste Blistervertiefung von der zweiten Blistervertiefung trennt;
wobei die internen Durchstechvorrichtungen jeweils ein Basiselement (210) und eine längliche Durchstechspitze (220) mit einem Kanal (230), der sich von dem Basiselement erstreckt, umfassen; und
wobei die erste interne Durchstechvorrichtung (120) der ersten Blistervertiefung konfiguriert ist, um eine Fluidverbindung mit einer externen Treibmittelquelle bereitzustellen, und die zweite interne Durchstechvorrichtung (140) der zweiten Blistervertiefung konfiguriert ist, um dem Anwender die trockene, pulverförmige Arzneimittelmischung bereitzustellen.

2. Dosierungsform (100) nach Anspruch 1, wobei die abgedichtete Grenzfläche, welche die erste Blistervertiefung (110) von der zweiten Blistervertiefung (130) trennt, konfiguriert ist, um einen Fluidverbindungsweg (150) von der ersten Blistervertiefung zu der zweiten Blistervertiefung bereitzustellen.

3. Dosierungsform (100) nach Anspruch 1, wobei mindestens eine der internen Durchstechvorrichtungen ferner eine ebene Dichtfläche (240) umfasst, die sich oberhalb des Basiselements und unterhalb des Endes der Durchstechspitze befindet.

4. Dosierungsform (100) nach Anspruch 3, wobei sich die ebene Dichtfläche (240) an der internen Durchstechvorrichtung der ersten Blistervertiefung (110) befindet, die konfiguriert ist, um eine Fluidverbindung mit einer externen Treibmittelquelle bereitzustellen.

5. Dosierungsform (100) nach Anspruch 1, wobei mindestens eine der internen Durchstechvorrichtungen ferner eine längliche Durchstechspitze (220) umfasst, die relativ zu der Mitte ihres Basiselements (210) seitlich versetzt ist.

6. Dosierungsform (100) nach Anspruch 1, wobei die längliche Durchstechspitze (220) mindestens einer internen Durchstechvorrichtung mehr als einen Kanal (230) beinhaltet, der sich von dem Basiselement erstreckt.

7. Dosierungsform (100) nach Anspruch 1, wobei der Kanal (230) der länglichen Durchstechspitze mindestens einer der internen Durchstechvorrichtungen aus einem ausgeschnittenen Kanal (230) besteht; und/oder wobei der Kanal (230) der länglichen Durchstechspitze mindestens einer der internen Durchstechvorrichtungen drehbar an dem Basiselement (210) konfiguriert ist, um einen gerichteten Strömungsweg bereitzustellen.

8. Dosierungsform (100) nach Anspruch 5, wobei der Kanal (230) der internen Durchstechvorrichtung relativ zu der Mitte ihres Basiselements (210) seitlich und gegenüber dem Ende ihrer Blistervertiefung, an dem das Treibmittel während der Abgabe einströmt, versetzt ist.

9. Freisetzungssystem (500) zur Verabreichung einer trockenen, pulverförmigen Arzneimittelmischung an ein Subjekt, wobei das Freisetzungssystem Folgendes umfasst:
eine handgeführte Abgabevorrichtung (400), die mehr als einen Kolben (410, 420, 430) umfasst;
eine innerhalb der handgeführten Abgabevorrichtung angeordnete Dosierungsform (100), die eine erste Blistervertiefung (110), die eine erste interne Durchstechvorrichtung (120) enthält, eine zweite Blistervertiefung (130), die eine zweite interne Durchstechvorrichtung (140) enthält, und einen Foliendeckel (104) umfasst, der eine abgedichtete Grenzfläche ausbildet, welche die erste Blistervertiefung von der zweiten Blistervertiefung trennt; und
wobei ein erster Kolben (410) der handgeführten Abgabevorrichtung konfiguriert ist, um die erste Blistervertiefung (110) der Dosierungsform zusammenzudrücken, um eine Fluidverbindung mit einer externen Treibmittelquelle (434) bereitzustellen, und ein zweiter Kolben (420) konfiguriert ist, um die zweite Blistervertiefung (130) der Dosierungsform zusammenzudrücken, um dem Anwender die trockene, pulverförmige Arzneimittelmischung bereitzustellen.

10. Freisetzungssystem (500) nach Anspruch 9, wobei die internen Durchstechvorrichtungen (120, 140) jeweils ein Basiselement (210) und eine längliche Durchstechspitze (220) mit einem Kanal (230), der sich von dem Basiselement erstreckt, umfassen.

11. Freisetzungssystem (500) nach Anspruch 9, wobei die handgeführte Abgabevorrichtung (400) ferner einen dritten Kolben (430) umfasst, um das externe Treibmittel an der ersten Blistervertiefung (110) bereitzustellen; und/oder wobei die handgeführte Abgabevorrichtung ferner eine elektromechanische Vorrichtung umfasst, um das externe Treibmittel (300) an der ersten Blistervertiefung bereitzustellen; und/oder wobei die handgeführte Abgabevorrichtung (400) ferner einen Mechanismus umfasst, um das Zusammendrücken der Blistervertiefungen zeitlich aufeinanderfolgend zu steuern.

12. Freisetzungssystem nach Anspruch 9, wobei das gasförmige Treibmittel (300) Stickstoff ist.

13. Freisetzungssystem (500) nach Anspruch 9, wobei eine längliche Durchstechspitze (220) mindestens einer der internen Durchstechvorrichtungen ferner eine ebene Dichtfläche (240) umfasst, die aus einem verbreiterten Teilabschnitt des Körpers der länglichen Durchstechspitze (220) ausgebildet ist, der sich oberhalb des Basiselements (210) und unterhalb des Endes der Durchstechspitze befindet.

## Revendications

1. Forme posologique unitaire (100) pour l'administration d'un composé médicinal en poudre sèche à un sujet, la forme posologique comprenant :
une première cavité de blister (110) contenant un premier dispositif de perforation interne (120) ;
une seconde cavité de blister (130) contenant un second dispositif de perforation interne (140) ;
un opercule (104) qui forme une interface étanche séparant la première cavité de blister de la seconde cavité de blister ;
dans laquelle les dispositifs de perforation interne comprennent chacun un élément de base (210) et une pointe de perforation allongée (220) avec un canal (230) s'étendant depuis l'élément de base ; et
dans laquelle le premier dispositif de perforation interne (120) de la première cavité de blister est configuré pour fournir une communication fluidique avec une source de propulseur externe et le second dispositif de perforation interne (140) de la seconde cavité de blister est configuré pour fournir le composé médicinal en poudre sèche à l'utilisateur.

2. Forme posologique (100) de la revendication 1, dans laquelle l'interface étanche séparant la première cavité de blister (110) de la seconde cavité de blister (130) est configurée pour fournir un chemin de communication fluidique (150) de la première cavité de blister à la seconde cavité de blister.

3. Forme posologique (100) de la revendication 1, dans laquelle au moins l'un des dispositifs de perforation interne comprend en outre une surface d'étanchéité plane (240) située au-dessus de l'élément de base et en dessous de l'extrémité de la pointe de perforation.

4. Forme posologique (100) de la revendication 3, dans laquelle la surface d'étanchéité plane (240) est située sur le dispositif de perforation interne de la première cavité de blister (110) configurée pour fournir une communication fluidique avec une source de propulseur externe.

5. Forme posologique (100) de la revendication 1, dans laquelle au moins l'un des dispositifs de perforation interne comprend en outre une pointe de perforation allongée (220) qui est décalée latéralement par rapport au centre de son élément de base (210).

6. Forme posologique (100) de la revendication 1, dans laquelle la pointe de perforation allongée (220) d'au moins un dispositif de perforation interne comporte plus d'un canal (230) s'étendant depuis l'élément de base.

7. Forme posologique (100) de la revendication 1, dans laquelle le canal (230) de la pointe de perforation allongée d'au moins l'un des dispositifs de perforation interne comporte un canal de type coupé (230) ; et/ou dans laquelle le canal (230) de la pointe de perforation allongée d'au moins l'un des dispositifs de perforation interne est configuré de manière rotative sur l'élément de base (210) pour fournir un chemin d'écoulement directionnel.

8. Forme posologique (100) de la revendication 5, dans laquelle le canal (230) du dispositif de perforation interne est décalé latéralement par rapport au centre de son élément de base (210) et est opposé à l'extrémité de sa cavité de blister où le propulseur s'écoule dans la cavité pendant la distribution.

9. Système d'administration (500) pour l'administration d'un composé médicinal en poudre sèche à un sujet, le système d'administration comprenant :
un dispositif de distribution portatif (400) comprenant plus d'un piston (410, 420, 430) ;
une forme posologique (100) disposée dans le dispositif de distribution portatif comprenant une première cavité de blister (110) contenant un premier dispositif de perforation interne (120), une seconde cavité de blister (130) contenant un second dispositif de perforation interne (140), et un opercule (104) qui forme une interface étanche séparant la première cavité de blister de la seconde cavité de blister ; et
dans lequel un premier piston (410) du dispositif de distribution portatif est configuré pour comprimer la première cavité de blister (110) de la forme posologique afin de fournir une communication fluidique avec une source de propulseur externe (434) et un deuxième piston (420) est configuré pour comprimer la seconde cavité de blister (130) de la forme posologique afin de fournir le composé médicinal en poudre sèche à l'utilisateur.

10. Système d'administration (500) de la revendication 9, dans lequel les dispositifs de perforation interne (120, 140) comprennent chacun un élément de base (210) et une pointe de perforation allongée (220) avec un canal (230) s'étendant depuis l'élément de base.

11. Système d'administration (500) de la revendication 9, dans lequel le dispositif de distribution portatif (400) comprend en outre un troisième piston (430) pour fournir le propulseur externe à la première cavité de blister (110) ; et/ou dans lequel le dispositif de distribution portatif comprend en outre un dispositif électromécanique pour fournir le propulseur externe (300) à la première cavité de blister ; et/ou dans lequel le dispositif de distribution portatif (400) comprend en outre un mécanisme pour synchroniser la compression des cavités de blister.

12. Système d'administration de la revendication 9, dans lequel le propulseur gazeux (300) est de l'azote.

13. Système d'administration (500) de la revendication 9, dans lequel une pointe de perforation allongée (220) d'au moins l'un des dispositifs de perforation interne comprend en outre une surface d'étanchéité plane (240) formée à partir d'une section élargie du corps de la pointe de perforation allongée (220) située au-dessus de l'élément de base (210) et en dessous de l'extrémité de la pointe de perforation.
